# FASCICULE DE BREVET EUROPEEN

(11) **EP 0 685 221 B1**
(45) Date de publication et mention de la délivrance du brevet: **30.06.1999**
(21) Numéro de dépôt: 95401055.9
(22) Date de dépôt: 05.05.1995
(51) Int. Cl.: A61K 7/42

(54) **Compositions cosmétiques photoprotectrices à base d'un mélange synergétique de filtres et utilisations**
Kosmetische Sonnenschutzmittel enthaltend eine synergistische Mischung aus Filtern und Verwendungen
Cosmetic sunscreening compositions containing a synergic mixture of filters and uses

(30) Priorité: 03.06.1994 FR 9406828
(43) Date de publication de la demande: 06.12.1995
(73) Titulaire: L'OREAL, 75008 Paris (FR)
(72) Inventeur: Ascione, Jean-Marc, F-75018 Paris (FR); Allard, Delphine, F-92700 Colombes (FR)
(74) Mandataire: Andral, Christophe André Louis

(56) Documents cités:
- EP-A- 0 457 687
- EP-A- 0 518 772

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association d'au moins deux filtres particuliers et convenablement sélectionnés, à savoir d'une part la 2,4,6-tris[p-(2'-éthylhexyl-1'-oxycarbonyl)anilino]-1,3,5-triazine et, d'autre part, l'α-cyano-β,β-diphénylacrylate de 2-éthylhéxyle, cette association conférant auxdites compositions, par un effet de synergie, des facteurs de protection solaire améliorés.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber selectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Or, à la suite d'importantes recherches menées dans le domaine de la photoprotection évoqué ci-dessus, la Demanderesse a découvert, de façon inattendue et surprenante, qu'une combinaison de deux filtres solaires particuliers et déja connus en soi dans l'état de l'art, permettait, du fait d'un effet de synergie, d'obtenir des compositions antisolaires présentant des indices de protection nettement améliorés, et en tous cas supérieurs à ceux qui peuvent être obtenus, à concentration égale de filtre et à nature identique de support, avec l'un ou l'autre des filtres utilisé seul.

Cette découverte est à la base de la présente invention.

Ainsi, conformément à l'un des objets de la présente invention, il est maintenant proposé de nouvelles compositions cosmétiques, en particulier antisolaires, qui sont essentiellement caractérisées par le fait qu'elles comprennent, dans un support cosmétiquement acceptable, (i) de la 2,4,6-tris[p-(2'-éthylhexyl-1'-oxycarbonyl)anilino]-1,3,5-triazine, à titre de premier filtre, et (ii) de l'α-cyano-β,β-diphénylacrylate de 2-éthylhéxyle à titre de second filtre.

La présente invention a également pour objet l'utilisation de telles compositions comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

D'autres caractéristiques, aspects et avantages de la présente invention apparaitront à la lecture de la description détaillée qui va suivre.

Comme indiqué précédemment, la 2,4,6-tris[p-(2'-éthylhexyl-1'-oxycarbonyl)anilino]-1,3,5-triazine (composé A) est un filtre connu en soi, actif dans l'UV-B et se présentant sous une forme solide, qui est vendu notamment sous la dénomination commerciale de "UVINUL T 150" par la Société BASF. Ce produit répond à la formule (I) suivante : 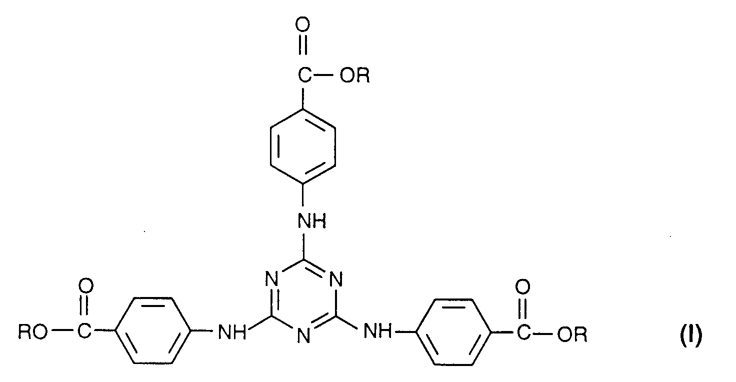 dans laquelle R désigne un radical 2-éthyl hexyle.

De même, l'α-cyano-β,β-diphénylacrylate de 2-éthylhéxyle (composé B), encore appelé octocrylène, est un filtre lipophile liquide déja connu en soi pour son activité également dans l'UV-B. Il s'agit d'un produit qui est disponible commercialement, et est vendu notamment sous la dénomination de "UVINUL N 539" par la Société BASF. Il répond à la formule (II) suivante : 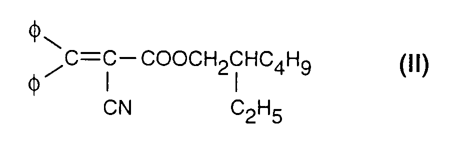 dans laquelle φ désigne un radical phényle.

Le composé A peut être présent dans les compositions selon l'invention à une concentration comprise entre 0,1 et 10 % en poids, et de préférence entre 0,5 et 5 % en poids, par rapport au poids total de la composition et le composé B peut être, quant à lui, présent à des teneurs comprises entre 0,5 et 15 % en poids, et de préférence entre 1 et 10 % en poids, toujours par rapport au poids total de la composition ; de préférence, la teneur globale du mélange entre le composé A et le composé B n'excède pas 15 % du poids total de la composition finale.

D'un point de vue pratique, les deux composés A et B ci-dessus sont bien entendu de préférence tous deux présents dans la composition finale dans des proportions respectives choisies de manière telle que l'effet de synergie, au niveau de l'indice de protection conféré par l'association résultante, soit optimal. La plage exacte des rapports pondéraux [composé A/composé B] dans laquelle cet effet de synergie optimal est effectivement atteint peut varier légèrement selon la quantité totale de filtres A et B mise en oeuvre.

En outre, et d'une manière générale, on notera que les concentrations et rapports en composés A et B sont choisis de manière telle que l'indice de protection solaire de la composition finale soit de préférence d'au moins 2.

Enfin, toujours selon un mode préféré de réalisation de la présente invention, le support cosmétiquement acceptable dans lequel se trouvent contenus les différents composés A et B, est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les deux filtres mentionnés ci-avant. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A- 0 518 772 et EP-A- 0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants et notamment les antioxydants ARL, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs. Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95 % en poids, de préférence de 70 à 90 % en poids, par rapport à l'ensemble de la formulation, la phase huileuse (comprenant notamment les filtres lipophiles) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

### EXEMPLE 1

On a préparé diverses formulations antisolaires se présentant sous la forme d'une émulsion de type huile-dans-eau et contenant (les quantités sont exprimées en % poids par rapport au poids total de la composition) :

| | |
|---|---|
| a) un premier filtre A qui était de la 2,4,6-tris[p-(2'-éthylhexyl-1'-oxycarbonyl)anilino]-1,3,5-triazine ("UVINUL T150"),à raison de | x % |
| b) un deuxième filtre B qui était l'α-cyano-β,β-diphénylacrylate de 2-éthylhéxyle ("UVINUL N 539"), à raison de | y % |
| c) Adipate de diisopropyle (corps gras) | 15% ou 20% |
| d) Terpolymère réticulé: acide méthacrylique/acrylate d'éthyle/steareth-10 allyl ether, en émulsion aqueuse à 30 % ("Salcare SC 90" de chez Allied Colloids)(émulsionnant) | 5 % |
| e) Triéthanolamine | 0,75 % |
| f) Conservateurs | qs |
| h) Eau | qsp 100 % |

ces formulations (numérotées 1 et 2 pour celles conformes à l'invention) présentant des rapports pondéraux y/x entre les filtres B et A différents ; à chacune de ces formulations, on a fait en outre correspondre des formulations comparatives qui soit ne contenaient que le filtre A à la concentration pondérale x, soit ne contenaient que le filtre B à la concentration pondérale y. Les formulations 1, 1A et 1B contenaient 20 % de corps gras, et les formulations 2, 2A et 2B seulement 15 % de corps gras.

On a réalisé chacune de ces émulsions en dissolvant les filtres dans la phase grasse, puis en ajoutant les émulsionnants dans cette phase grasse portée aux environs de 80°C, et enfin en additionnant sous agitation rapide la phase aqueuse préalablement chauffée à cette même température.

Pour chacune des formulations ainsi préparées, on a ensuite déterminé le facteur de protection solaire (SPF) qui leur était attaché. Celui-ci a été déterminé en utilisant la méthode *in vitro* décrite par B.L. DIFFEY et al. dans J. Soc. Cosmet. Chem. 40-127-133 (1989) ; cette méthode consiste à déterminer les facteurs de protection monochromatiques tous les 5 nm dans une gamme de longueurs d'onde de 290 à 400 nm et à calculer à partir de ceux-ci le facteur de protection solaire selon une équation mathématique donnée.

Les compositions des différentes formulations étudiées et les résultats en facteur de protection moyen obtenus sont rassemblés dans le tableau I donné ci-dessous.

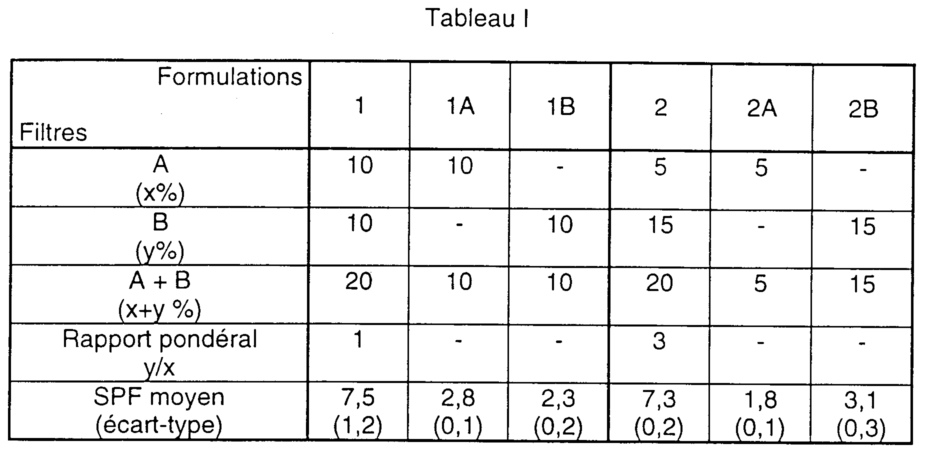

Ces résultats démontrent clairement l'effet de synergie obtenu avec les compositions 1 et 2 conformes à l'invention, les facteurs de protection solaire attachés à ces dernières étant dans tous les cas notablement et significativement supérieurs à la simple somme arithmétique des facteurs de protection solaire des compositions comparatives correspondantes ne contenant qu'un seul filtre.

### EXEMPLE 2

On donne ci-dessous un exemple concret de composition antisolaire conforme à l'invention se présentant sous la forme d'une émulsion de type huile-dans-eau.

| | |
|---|---|
| - 2,4,6-tris[p-(2'-éthylhexyl-1'-oxycarbonyl)anilino]-1,3,5-triazine ("UVINUL T150") | 4 g |
| - α-cyano-β, β-diphénylacrylate de 2-éthylhéxyle ("UVINUL N 539") | 10 g |
| - p-méthoxycinnamate de 2-éthylhexyle ("PARSOL MCX" de chez Givaudan)(filtre solaire) | 1 g |
| - Mélange d'alcool cétylstéarylique et d'alcool cétylstéarylique oxyéthyléné à 33 moles d'oxyde d'éthylène ("SINNOWAX AO" de chez Henkel)(émulsionnant) | 7 g |
| - Mélange de mono- di et tristéarate de glycérol (co-émulsionnant) | 2 g |
| - Adipate de diisopropyle | 15 g |
| - Polydiméthysiloxane | 1,5 g |
| - Alcool cétylique | 1,5 g |
| - Conservateurs | qs |
| - Eau distillée | qsp 100 g |

Cette émulsion a été préparée comme à l'exemple 1.

### EXEMPLE 3

On donne ci-dessous un autre exemple concret de composition antisolaire conforme à l'invention se présentant cette fois sous la forme d'une émulsion de type eau-dans-huile.

| | |
|---|---|
| - 2,4,6-tris[p-(2'-éthylhexyl-1'-oxycarbonyl)anilino]-1,3,5-triazine ("UVINUL T150") | 1 g |
| - α-cyano-β,β-diphénylacrylate de 2-éthylhéxyle ("UVINUL N 539") | 5 g |
| - Mélange [hydroxystéarate et isostéarate de sorbitol et de glycérol] à 20 moles d'oxyde de propylène et 30 moles d'oxyde d'éthylène ("ARLACEL 780" de chez ICI) | 2,5 g |
| - NaCl | 0,7 g |
| - Huile de vaseline | 20 g |
| - Conservateurs | qs |
| - Parfum | qs |
| - Eau | qsp 100 g |

## Revendications

1. Compositions cosmétiques à usage topique, en particulier pour la photoprotection de la peau et/ou des cheveux, caractérisées par le fait qu'elles comprennent, dans un support cosmétiquement acceptable, (i) de la 2,4,6-tris[p-(2'-éthylhexyl-1'-oxycarbonyl)anilino]-1,3,5-triazine, à titre de premier filtre, et (ii) de l'α-cyano-β,β-diphénylacrylate de 2-éthylhéxyle à titre de second filtre.

2. Compositions selon la revendication 1, caractérisées par le fait que ledit premier filtre est présent à une teneur comprise entre 0,1 et 10 % en poids par rapport au poids total de la composition.

3. Compositions selon la revendication 2, caractérisées par le fait que ladite teneur est comprise entre 0,5 et 5 % en poids.

4. Compositions selon l'une quelconque des revendications précédentes, caractérisées par le fait que ledit second filtre est présent à une teneur comprise entre 0,5 et 15 % en poids par rapport au poids total de la composition.

5. Compositions selon la revendication 4, caractérisées par le fait que ladite teneur est comprise entre 1 et 10 % en poids.

6. Compositions selon l'une quelconque des revendications précédentes, caractérisées par le fait que ledit support cosmétiquement acceptable se présente sous la forme d'une émulsion de type huile-dans-eau.

7. Compositions selon l'une quelconque des revendications précédentes, caractérisées par le fait qu'elles comprennent en outre un ou plusieurs filtres organiques complémentaires actifs dans l'UV-A et/ou UV-B, hydrophiles ou lipophiles.

8. Compositions selon la revendication 7, caractérisées par le fait que lesdits filtres organiques complémentaires sont choisis parmi les dérivés cinnamiques, les dérivés salicyliques, les dérivés du camphre, les dérivés de triazine, les dérivés de la benzophénone, les dérivés du dibenzoylméthane, les dérivés de β,β -diphénylacrylate, les dérivés de l'acide p-aminobenzoïque, les polymères filtres et silicones filtres

9. Compositions selon l'une quelconque des revendications précédentes, caractérisées par le fait qu'elles comprennent en outre, à titre d'agents photoprotecteurs complémentaires, des pigments ou des nanopigments d'oxydes métalliques, enrobés ou non, capables de bloquer physiquement, par diffusion et/ou réflection, le rayonnement UV.

10. Compositions selon la revendication 9, caractérisées par le fait que lesdits pigments ou nanopigments sont choisis parmi les oxydes de titane, de zinc, de fer, de zirconium, de cérium et leurs mélanges, enrobés ou non.

11. Compositions selon l'une quelconque des revendications précédentes, caractérisées par le fait qu'elles comprennent en outre au moins un agent de bronzage et/ou de brunissage artificiel de la peau.

12. Compositions selon l'une quelconque des revendications précédentes, caractérisées par le fait qu'elles comprennent en outre au moins un adjuvant choisi parmi les corps gras, les solvants organiques, les épaississants ioniques ou non ioniques, les adoucissants, les antioxydants et notamment les antioxydants ARL, les opacifiants, les stabilisants, les émollients, les silicones, les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

13. Compositions selon l'une quelconque des revendications précédentes, caractérisées par le fait qu'il s'agit de compositions protectices de l'épiderme humain ou de compositions antisolaires et qu'elles se présentent sous forme de dispersions vésiculaires non ioniques, émulsions, en particulier émulsions de type huile-dans-eau, crèmes, laits, gels, gels crèmes, suspensions, dispersions, poudres, bâtonnets solides, mousses ou spray.

14. Compositions selon l'une quelconque des revendications 1 à 12, caractérisées par le fait qu'il s'agit de compositions de maquillage des cils, des sourcils ou de la peau et qu'elles se présentent sous forme solide ou pâteuse, anhydre ou aqueuse, d'émulsions, de suspensions ou de dispersions.

15. Compositions selon l'une quelconque des revendications 1 à 12, caractérisées par le fait qu'il s'agit de compositions destinées à la protection des cheveux contre les rayons ultraviolets et qu'elles se présentent sous forme de shampooings, de lotions, de gels, d'émulsions, de dispersions vésiculaires non ioniques ou de laques pour cheveux.

16. Compositions selon l'une quelconque des revendications précédentes, caractérisées par le fait qu'elles présentent un indice de protection sur peau d'au moins 2.

17. Utilisation des compositions définies à l'une quelconque des revendications précédentes comme, ou pour la fabrication de, compositions cosmétiques pour la protection de la peau et/ou des cheveux contre le rayonnement ultraviolet, en particulier le rayonnement solaire.

18. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 16.

## Claims

1. Cosmetic compositions for topical use, in particular for the photoprotection of the skin and/or the hair, characterized in that they comprise, in a cosmetically acceptable support, (i) 2,4,6-tris[p-((2'-ethylhexyl)oxycarbonyl)anilino]-1,3,5-triazine, as first screening agent, and (ii) 2-ethylhexyl α-cyano-β,β-diphenylacrylate as second screening agent.

2. Compositions according to Claim 1, characterized in that the said first screening agent is present at a content between 0.1 and 10 % by weight relative to the total weight of the composition.

3. Compositions according to Claim 2, characterized in that the said content is between 0.5 and 5 % by weight.

4. Compositions according to any one of the preceding claims, characterized in that the said second screening agent is present at a content between 0.5 and 15 % by weight relative to the total weight of the composition.

5. Compositions according to Claim 4, characterized in that the said content is between 1 and 10 % by weight.

6. Compositions according to any one of the preceding claims, characterized in that the said cosmetically acceptable support is in the form of an emulsion of oil-in-water type.

7. Compositions according to any one of the preceding claims, characterized in that they additionally comprise one or more additional hydrophilic or lipophilic organic screening agents active in the UV-A and/or UV-B range.

8. Compositions according to Claim 7, characterized in that the said additional organic screening agents are chosen from cinnamic derivatives, salicylic derivatives, camphor derivatives, triazine derivatives, benzophenone derivatives, dibenzoylmethane derivatives, β,β-diphenylacrylate derivatives, p-aminobenzoic acid derivatives, screening polymers and screening silicones.

9. Compositions according to any one of the preceding claims, characterized in that they additionally comprise, as additional photoprotective agents, pigments or nanopigments of coated or noncoated metal oxides, capable of physically blocking the UV radiation, by diffusion and/or reflection.

10. Compositions according to Claim 9, characterized in that the said pigments or nanopigments are chosen from titanium oxide, zinc oxide, iron oxide, zirconium oxide, cerium oxide and mixtures thereof, which may or may not be coated.

11. Compositions according to any one of the preceding claims, characterized in that they additionally comprise at least one agent for the artificial tanning and/or browning of the skin.

12. Compositions according to any one of the preceding claims, characterized in that they additionally comprise at least one adjuvant chosen from fats, organic solvents, ionic or nonionic thickening agents, softeners, antioxidants and especially anti-free-radical antioxidants, opacifying agents, stabilizing agents, emollients, silicones, α-hydroxy acids, anti-foaming agents, hydrating agents, vitamins, fragrances, preserving agents, surfactants, fillers, sequestering agents, polymers, propellants, basifying or acidifying agents and dyes.

13. Compositions according to any one of the preceding claims, characterized in that they are compositions for protecting the human epidermis or antisun compositions and in that they are in the form of nonionic vesicle dispersions, emulsions, in particular emulsions of oil-in-water type, creams, milks, gels, cream gels, suspensions, dispersions, powders, solid sticks, foams or spray.

14. Compositions according to any one of Claims 1 to 12, characterized in that they are compositions for making up the eyelashes, the eyebrows or the skin and in that they are in anhydrous or aqueous, solid or pasty form, emulsion form, suspension form or dispersion form.

15. Compositions according to any one of Claims 1 to 12, characterized in that they are compositions intended for the protection of the hair against ultraviolet rays and in that they are in the form of shampoos, lotions, gels, emulsions, nonionic vesicle dispersions or lacquers for the hair.

16. Compositions according to any one of the preceding claims, characterized in that they have a protection factor on the skin of at least 2.

17. Use of the compositions defined in any one of the preceding claims as, or for the manufacture of, cosmetic compositions for the protection of the skin and/or the hair against ultraviolet radiation, in particular solar radiation.

18. Cosmetic treatment process for protecting the skin and/or the hair against ultraviolet radiation, in particular solar radiation, characterized in that it consists in applying an effective amount of a composition as defined in any one of Claims 1 to 16 to the skin and/or the hair.

## Patentansprüche

1. Kosmetische Zusammensetzungen zur topischen Anwendung, insbesondere zum Lichtschutz der Haut und/oder der Haare, dadurch gekennzeichnet, daß sie in einem kosmetisch akzeptablen Träger enthalten:
(i) 2,4,6-Tris[p-(2'-ethylhexyl-1'-oxycarbonyl)-anilino]-1,3,5-triazin als erstes Filter
und
(ii) 2-Ethylhexyl-α-cyano-β,β-diphenylacrylat als zweites Filter.

2. Zusammensetzungen nach Anspruch 1, dadurch gekennzeichnet, daß das erste Filter in einem Mengenanteil von 0,1 bis 10 Gew.-%, bezogen auf das Gesamtgewicht der Zusammensetzung, vorliegt.

3. Zusammensetzungen nach Anspruch 2, dadurch gekennzeichnet, daß der Mengenanteil 0,5 bis 5 Gew.-% beträgt.

4. Zusammensetzungen nach einem der vorhergehenden Ansprüche, dadurch gekennzeichnet, daß das zweite Filter in einem Mengenanteil von 0,5 bis 15 Gew.-%, bezogen auf das Gesamtgewicht der Zusammensetzung, vorliegt.

5. Zusammensetzungen nach Anspruch 4, dadurch gekennzeichnet, daß der Mengenanteil 1 bis 10 Gew.-% beträgt.

6. Zusammensetzungen nach einem der vorhergehenden Ansprüche, dadurch gekennzeichnet, daß der kosmetisch akzeptable Träger in Form einer Emulsion vom Öl-in-Wasser-Typ vorliegt.

7. Zusammensetzungen nach einem der vorhergehenden Ansprüche, dadurch gekennzechnet, daß sie ferner ein oder mehrere zusätzliche hydrophile oder lipophile organische Filter enthalten, die im UV-A-Bereich und/oder im UV-B-Bereich wirksam sind.

8. Zusammensetzungen nach Anspruch 7, dadurch gekennzeichnet, daß die zusätzlichen organischen Filter ausgewählt sind unter Zimtsäurederivaten, Salicylsäurederivaten, Campherderivaten, Triazinderivaten, Benzophenonderivaten, Dibenzoylmethanderivaten, β,β-Diphenylacrylat-Derivaten, Derivaten der p-Aminobenzoesäure, polymeren Filtern und Siliconfiltern.

9. Zusammensetzungen nach einem der vorhergehenden Ansprüche, dadurch gekennzeichnet, daß sie ferner als zusätzliche Lichtschutzmittel Pigmente oder Nanopigmente von Metalloxiden enthalten, die gegebenenfalls ummantelt sind und befähigt sind, UV-Strahlung durch Diffusion und/oder Reflexion physikalisch zu blockieren.

10. Zusammensetzungen nach Anspruch 9, dadurch gekennzeichnet, daß die Pigmente oder Nanopigmente unter Oxiden von Titan, Zink, Eisen, Zirconium und Cer sowie deren Gemischen ausgewählt und gegebenenfalls ummantelt sind.

11. Zusammensetzungen nach einem der vorhergehenden Ansprüche, dadurch gekennzeichnet, daß sie ferner mindestens ein Mittel zur künstlichen Bräunung und/oder Braunfärbung der Haut enthalten.

12. Zusammensetzungen nach einem der vorhergehenden Ansprüche, dadurch gekennzeichnet, daß sie ferner mindestens einen Hilfsstoff enthalten, der ausgewählt ist unter Fettstoffen, organischen Lösungsmitteln, ionischen oder nichtionischen Verdickungsmitteln, Mitteln für die Geschmeidigkeit, Antioxidationsmitteln und insbesondere ARL-Antioxidationsmitteln, Trübungsmitteln, Stabilisierungsmitteln, Emollientien, Siliconen, α-Hydroxysäuren, Antischaummitteln, Hydratisierungsmitteln, Vitaminen, Parfums, Konservierungsmitteln, grenzflächenaktiven Mitteln, Füllstoffen, Maskierungsmitteln, Polymeren, Treibmitteln, Mitteln zum Alkalischmachen, Ansäuerungsmitteln und Färbemitteln.

13. Zusammensetzungen nach einem der vorhergehenden Ansprüche, dadurch gekennzeichnet, daß es sich um Zusammensetzungen zum Schutz der menschlichen Epidermis oder Zusammensetzungen zum Sonnenschutz handelt und sie in Form von nichtionischen Vesikeldispersionen, Emulsionen, insbesondere Emulsionen vom Öl-in-Wasser-Typ, Cremen, Milchen, Gelen, Gelcremen, Suspensionen, Dispersionen, Pudern, festen Stiften, Schäumen oder Sprays vorliegen.

14. Zusammensetzungen nach einem der Ansprüche 1 bis 12, dadurch gekennzeichnet, daß es sich um Zusammensetzungen zum Schminken der Wimpern, der Augenbrauen oder der Haut handelt und sie in fester oder pastoser, wasserfreier oder wasserhaltiger Form als Emulsionen, Suspensionen oder Dispersionen vorliegen.

15. Zusammensetzungen nach einem der Ansprüche 1 bis 12, dadurch gekennzeichnet, daß es sich um Zusammensetzungen zum Schutz der Haare gegen Ultraviolettstrahlung handelt und sie in Form von Shampoos, Lotionen, Gelen, Emulsionen, nichtionischen Vesikeldispersionen oder Haarlacken vorliegen.

16. Zusammensetzungen nach einem der vorhergehenden Ansprüche, dadurch gekennzeichnet, daß sie einen Schutzfaktor auf der Haut von mindestens 2 aufweisen.

17. Verwendung der Zusammensetzungen nach einem der vorhergehenden Ansprüche als kosmetische Zusammensetzungen oder zur Herstellung von kosmetischen Zusammensetzungen zum Schutz der Haut und/oder der Haare gegen ultraviolette Strahlung, insbesondere Sonnenstrahlung.

18. Verfahren zur kosmetischen Behandlung zum Schutz der Haut und/oder der Haare gegen Ultraviolettstrahlung, insbesondere Sonnenstrahlung, dadurch gekennzeichnet, daß es in der Anwendung einer wirksamen Menge einer Zusammensetzung nach einem der Ansprüche 1 bis 16 auf die Haut und/oder die Haare besteht.
